# EUROPÄISCHE PATENTANMELDUNG

(11) **EP 1 430 891 A1**
(43) Veröffentlichungstag der Anmeldung: **23.06.2004**
(21) Anmeldenummer: 02028512.8
(22) Anmeldetag: 19.12.2002
(51) Int. Cl.: A61K 31/19, A61K 31/194, A61P 15/10, A61P 19/02, A61P 19/06

(54) **Öl-Säure-Emulsion zur Behandlung von Gelenkerkrankungen und zur Durchblutungsanregung**

(71) Anmelder: Fleischmann, Elisabeth, 85356 Freising (DE)
(72) Erfinder: Fleischmann, Elisabeth, 85356 Freising (DE)

(57) **Zusammenfassung**

Die vorliegende Erfindung betrifft Zubereitungen, die Öl, Emulgator und mindestens eine organische Säure, ausgewählt unter Essigsäure, Weinsäure, Zitronensäure enthalten und deren Verwendung zur Durchblutungsanregung und zur Behandlung von Gelenkerkrankungen. Weiterhin können erfindungsgemäße Zubereitungen zum Erzielen eines angenehm kühlenden Effekts bei einem Auftrag auf die Haut verwendet werden.

## Beschreibung

Die vorliegende Erfindung betrifft Zubereitungen, die Öl, Emulgator und mindestens eine organische Säure, ausgewählt unter Essigsäure, Weinsäure, Zitronensäure, enthalten, sowie deren Verwendung zur Behandlung von Gelenkerkrankungen und zur Durchblutungsanregung. Weiterhin können erfindungsgemäße Zubereitungen zum Erzielen eines angenehm kühlenden Effekts bei einem Auftrag auf der Haut verwendet werden.

Durchblutungsstörungen, insbesondere der Extremitäten und der Haut, sowie Gelenkerkrankungen, insbesondere entzündliche Gelenkerkrankungen, können unterschiedlichste und teilweise noch nicht vollständig geklärte Ursachen haben. Allen diesen Erkrankungen ist jedoch gemeinsam, dass sie erhebliche Schmerzen hervorrufen können und Betroffene in ihrer Bewegungsfähigkeit stark einschränken.

Aufgabe der vorliegenden Erfindung ist die Bereitstellung einer Zubereitung, welche sich sowohl bei gesunden Personen, als auch bei Personen, die von Durchblutungsstörungen oder Gelenkerkrankungen betroffen sind, wohltuend auswirkt.

Erfindungsgemäß wird diese Aufgabe durch ein Gemisch gelöst, welches Öl, Emulgator und mindestens eine organische Säure ausgewählt unter Essigsäure, Weinsäure, Zitronensäure enthält.

Bei geeignet gewählter Formulierungs- und Auftragungsweise tritt primär bei einem Auftrag eines erfindungsgemäßen Gemisches auf der Haut ein kühlender Effekt auf, der von den Benutzern als äußerst angenehm und wohltuend empfunden wird, beispielsweise im Falle einer Entzündung im Bereich der Gelenke oder bei einer Venenentzündung.

Neben diesem angenehmen und wohltuenden Effekt konnten darüber hinaus bei den zahlreichen Versuchen, die zu der Entwicklung der vorliegenden Erfindung führten, weitere überraschende, positive Wirkungen eines erfindungsgemäßen Gemisches beobachtet werden. Insbesondere kann ein erfindungsgemäßes Gemisch zur Behandlung von Gelenkerkrankungen oder zur Durchblutungsanregung verwendet werden.

So zeigte sich, dass ein erfindungsgemäßes Gemisch, beispielsweise beim Auftragen auf der und/oder Einmassieren in die Haut (oder bei einer anderen dem Fachmann bekannten Anwendungsweise) eine Anregung der Durchblutung und gegebenenfalls eine Lockerung der Muskulatur bewirken kann. Eine derartige Anregung der Durchblutung ist für vielfältige, einem Fachmann bekannte Anwendungsmöglichkeiten geeignet und ist bei vielen Körperzuständen und Erkrankungen hilfreich, wohltuend und heilungsfördernd. Ein erfindungsgemäßes Gemisch kann so beispielsweise nach Überbeanspruchungen des Körpers beim Sport oder bei leichten Sportverletzungen angewendet werden. Das erfindungsgemäße Gemisch kann auch genutzt werden, wenn trotz ausreichender Raumtemperatur ein Kältegefühl in den Gliedmaßen vorherrscht. In diesem Fall kann es eine Verringerung dieses Kältegefiihls bewirken. Besonders wichtig ist eine Förderung der Durchblutung und eine Anregung des Körpers bei amputationsgefährdeten Gliedmaßen. Hier kann durch Anwendung des erfindungsgemäßen Gemischs (z.B. durch Einreibung) eine Stabilisierung oder Verbesserung der Gesamtsituation des betroffenen Körperteils erreicht und eine Amputation verhindert werden.

Ebenso nutzbringend und überraschend sind die Wirkungen, welche bei einer äußerlichen Anwendung eines erfindungsgemäßen Gemisches auf der Haut über einem von Gicht betroffenen Körperbereich beobachtet werden können. So verringerte sich beispielsweise der Umfang eines von Gicht betroffenen Gelenks erkennbar und auch die Gicht-bedingten Schmerzen gingen stark oder sogar vollständig zurück.

Wie allgemein bekannt ist, handelt es sich bei Gicht um eine Purinstoffwechselstörung, die durch eine Abscheidung von harnsauren Salzen an verschiedenen Körperstellen, insbesondere in den Gelenken und in deren Umgebung charakterisiert ist. Eine mögliche Erklärung für die positiven Eigenschaften eines erfindungsgemäßen Gemisches ist, dass die in Emulsion vorliegende Essigsäure über die Haut in den Körper transportiert wird und zu einer Auflösung von Ablagerungen (insbesondere zu einer Auflösung von den dort abgelagerten harnsauren Salzen) und/oder zu deren Abtransport führt. Anwender erklärten, dass das erfindungsgemäße Gemisch derart wirke, als ob die "Kristalle aus der Haut herausgezogen würden". Neben den vorstehenden Erklärungen sind jedoch weitere Erklärungsmöglichkeiten für die Wirksamkeit des erfindungsgemäßen Gemisches möglich. Insbesondere wird nicht beabsichtigt, dass falls sich die vorstehenden Erläuterungen als unvollständig oder falsch herausstellen sollten, der Schutzumfang der Erfindung hierdurch in irgendeiner Weise eingeschränkt wird.

Eine erfindungsgemäße Verwendung des Gemisches kann jedoch nicht nur bei Gicht oder gichtartigen Erkrankungen, sondern auch bei Gelenkerkrankungen im Allgemeinen, und insbesondere bei entzündlichen Gelenkerkrankungen (Arthritis) und rheumatischen Erkrankungen, erfolgen. Darüber hinaus kann das erfindungsgemäße Gemisch auch zur Verringerung von Entzündungsreaktionen dienen. So tritt bei den vorstehend erwähnten Erkrankungen, beispielsweise nach einem Einreiben und/oder Einmassieren der Haut im betroffenen Bereich mit dem erfindungsgemäßen Gemisch, eine Schmerzlinderung, eine Verbesserung der Beweglichkeit und/oder eine Verbesserung des Zustands des erkrankten Bereichs auf.

Eine erfindungsgemäße Verwendung kann außerdem auch nach Verletzungen oder Überbeanspruchungen im Bereich der Gelenke, beispielsweise nach leichten Sportverletzungen, erfolgen, wobei auch hier von einer abschwellenden und schmerzlindernden Wirkung einer erfindungsgemäßen Zubereitung berichtet wurde.

Der Begriff "im Bereich der Gelenke" oder "im betroffenen Bereich", wie in der vorliegenden Erfindung verwendet, umfasst nicht nur den jeweils betroffenen gesamten Gelenkaufbau oder Bereich, sondern auch die umgebenden Gewebe, Muskeln, Sehnen, usw., sowohl in direkt angrenzenden, als auch in mehrere Zentimeter, beispielsweise bis zu 70 cm; häufiger in bis zu 10 cm, entfernten Gebieten.

Weitere überraschende, positive Wirkungen zeigten sich bei einem Einreiben des erfindungsgemäßen Gemisches im Kopf- und Nackenbereich. Nach einer derartigen Behandlung trat eine vollständige Behebung oder erhebliche Linderung von Kopfschmerzen auf, was möglicherweise durch eine deutliche Entspannung und/oder Verbesserung der Durchblutung im Bereich der Schläfen- und Nackenmuskulatur erklärt werden kann.

Das erfindungsgemäße Gemisch wirkt bei einer Verwendung zu einer Körper- oder Rückenmassage erektionsfördernd, insbesondere bei Stress, Erschöpfung oder "vorzeitigem Nachlassen". Auch hier könnten die beobachteten positiven Effekte über eine Veränderung und/oder Verbesserung der Durchblutung erklärt werden.

Darüber hinaus eignet sich das erfindungsgemäße Gemisch auch zur Behandlung von Hämorrhoiden oder zu einer Schmerzlinderung bei Hämorrhoiden.

Bei den vielfältigen positiven Wirkungen des erfindungsgemäßen Gemisch spielen neben den für einen Fachmann offensichtlichen Eigenschaften, wie pH-Wert oder Molekülgröße, auch bislang nicht geklärte, synergistische Wirkungen eine wichtige Rolle.

Die Essigsäure (und gegebenenfalls in dem Gemisch enthaltenes Wasser) kann bei einem erfindungsgemäßen Gemisch vollständig oder teilweise in Emulsion vorliegen, d.h. die vorliegende Erfindung umfasst sowohl Emulsionen, als auch mehrphasige Gemische, die erst unmittelbar vor der Verwendung, beispielsweise durch Schütteln oder Umrühren, miteinander vermischt werden.

Die zur Herstellung eines erfindungsgemäßen Gemisches gewählten Mengen werden in Abhängigkeit von der geplanten Anwendung bestimmt werden. Als vorteilhaft haben sich beispielsweise Gemische erwiesen, die 0,2 bis 3 Vol.-% an Essigsäure, bevorzugt 0,7 bis 2,4 Vol.-% an Essigsäure, 65 bis 90 Vol.-% an Öl, bevorzugt 75 bis 85 Vol.-% an Öl und 5 bis 14 Vol.-% an Emulgator, bevorzugt 6 bis 12 Vol.-% an Emulgator umfassen. Bei Gemischen mit den vorstehend aufgeführten Zusammensetzungen treten offensichtlich in besonderem Maße synergistische Effekte auf, die zu einer besonders guten Wirksamkeit führen.

Ähnlich vorteilhaft sind Gemische auf Basis von Zitronensäure oder Weinsäure, insbesondere Gemische, welche 0,2 bis 3 Gew.-% an Zitronensäure oder Weinsäure, bevorzugt 0,7 bis 2,4 Gew.-% an Zitronensäure oder Weinsäure, 65 bis 90 Vol.-% an Öl, bevorzugt 75 bis 85 Vol.-% an Öl und 5 bis 14 Vol.-% an Emulgator, bevorzugt 6 bis 12 Vol.-% an Emulgator umfassen. Natürlich können auch Zubereitungen hergestellt werden, die beliebige Kombinationen (d.h. beispielsweise Gemische) der vorstehend aufgeführten Gemische umfassen.

Neben den vorstehend angegebenen Substanzen kann ein erfindungsgemäßes Gemisch weitere, im Stand der Technik bekannte, geeignete Substanzen umfassen. Insbesondere wird das Gemisch Wasser umfassen, falls Essigsäure, Zitronensäure oder Weinsäure in Form einer wässrigen Lösung, beispielsweise in Form einer 10 - 25 Gew.-% der jeweiligen Säure(n) enthaltenden, wässrigen Lösung zugesetzt werden.

Ein erfindungsgemäßes Gemisch kann beispielsweise in Form einer Creme, eines Öls oder Badezusatzes bereitgestellt werden. Darüber hinaus ist jedoch auch jegliche andere, im Stand der Technik bekannte Zubereitungs- oder Formulierungsform verwendbar, insbesondere Zubereitungen oder Formulierungen zur topischen oder äußerlichen Anwendung, beispielsweise Sprayzubereitungen, Salben, Gele, Lotionen, adhäsive Zubereitungen, Zäpfchen oder Pflasterzubereitungen. Bei Formulierung eines Gemisches zur oralen Einnahme wird die jeweilige Säurekonzentration derart gewählt, dass die Zubereitung einen zur oralen Einnahme geeigneten pH-Wert aufweist. Als Emulgator wird nur ein verzehrbarer Emulgator eingesetzt, wobei gegebenenfalls auf den Zusatz von Emulgatoren verzichtet werden kann und vorzugsweise Vitamin E-acetat anstelle von Vitamin E verwendet wird.

Bei Verwendung in Form einer Creme oder eines Öls kann beispielsweise ein einmaliger oder wiederholter Auftrag, beispielsweise einmal oder mehrmals täglich in einem Zeitraum zwischen einem Tag und mehreren Monaten oder länger, auf der Haut im Bereich des betroffenen Körperbereichs oder großflächig über weite Bereiche des gesamten Körpers erfolgen. Bei einer Verwendung in Form eines Badezusatzes können beispielsweise zwischen 1 und 100 ml des erfindungsgemäßen Gemisches oder Badezusatzes auf ein übliches Vollbad zugegeben werden.

Zur Herstellung eines erfindungsgemäßen Gemisches können als "Öl" pflanzliche Öle, tierische Öle, mineralische Öle, Wachse und/oder Kombinationen hiervon verwendet werden.

Der Begriff "Öl", wie er im Rahmen der vorliegenden Erfindung verwendet wird, umfasst Stoffe beliebiger Konsistenz, d.h. auch Stoffe, die bei Raumtemperatur halbfest oder fest vorliegen. Aufgrund der einfacheren Handhabung werden jedoch im allgemeinen flüssige Öle bevorzugt.

Als pflanzliche Öle eignen sich beispielsweise Öle wie Avocadoöl, Sojaöl, Schwarzkümmelöl, Traubenkernöl, Olivenöl, Palmöl, Sonnenblumenöl, Erdnußöl, Rapsöl, Sesamöl, Weizenkeimöl und Mandelöl. Als mineralische Öle kommen insbesondere sog. Paraffinöle, aber auch einzelne Paraffine, und Paraffine oder Paraffingemische umfassende organische Substanzgemische in Betracht. Weiterhin können auch beliebige tierische Fette, beispielsweise Seetieröle, Produkte auf Basis von Milchfett oder Schweineschmalz verwendet werden. Darüber hinaus können, als Öl auch Mazerate oder Ölauszüge von pflanzlichen Bestandteilen auf Basis eines beliebigen pflanzlichen, tierischen oder mineralischen Öls, beispielsweise im Handel oder in der Apotheke erhältliches Johanniskraut-Mazerate oder sog. Ringelblumenöl verwendet werden.

Wie einem Fachmann klar ist, kann das zur Herstellung einer erfindungsgemäßen Zubereitung verwendete Öl teilweise oder vollständig durch Mono-, Di- und/oder Triacylglyceride umfassende Gemische ersetzt werden. Darüber hinaus kann das Öl auch teilweise oder vollständig durch Wachse, beispielsweise Bienenwachs oder bei Raumtemperatur flüssige Wachse, oder durch Fettalkohol und/oder Fettalkohol-Fettsäureester umfassende Gemische, ersetzt werden. Darüber hinaus können natürlich auch beliebige Kombinationen der vorstehend aufgeführten Öle oder Öl-verwandten Produkte zur Herstellung einer erfindungsgemäßen Zubereitung verwendet werden.

Bei der Herstellung des erfindungsgemäß zu verwendenden Gemisches können neben reiner oder im wesentlichen reiner Essigsäure oder wässrigen Essigsäurelösungen, wie beispielsweise handelsüblicher, sog. Essigessenz, auch beliebige im Stand der Technik bekannte Essigsorten, wie Weinessig oder Apfelessig verwendet werden. Weinsäure und Zitronensäure können in reiner oder in einer im wesentlichen reinen Form oder vorteilhafterweise in Form von wässrigen Lösungen eingesetzt werden. Natürlich können neben den freien Säuren auch Verbindungen eingesetzt werden aus denen Essigsäure, Weinsäure oder Zitronensäure freisetzbar sind, beispielsweise Säuresalze oder Säure-Depotverbindungen, insbesondere in Kombinationen mit beliebigen, eine Säure-Freisetzung unterstützenden Substanzen.

Zur Gewinnung des erfindungsgemäßen Gemisches können beliebige, im Stand der Technik bekannte, für eine Anwendung im kosmetischen, medizinischen oder pharmazeutischen Bereich geeignete Emulgatoren, verwendet werden. Beispielsweise eignen sich Emulgatoren auf Basis von PEG (Polyethylenglykol) - Derivaten, insbesondere PEG-40 Rizinusöl (PEG-40 Castor Oil), PEG-40 hydriertes Rizinusöl (PEG-40 Hydrogenated Castor Oil), PEG-40 Stearat (PEG-40 Stearate), Phospholipide, Vitamin E (α-Tocopherol), Tocopherole, Tocopherylacetate, Tocopherol-Vorläuferverbindungen und/oder Tocopherol-Derivate, beispielsweise Verbindungen, die das Tocol (2-Methyl-2-(4',8',12'-trimethyl-tridecyl)-chroman-6-ol)-Gerüst aufweisen. Natürlich können auch beliebige Kombinationen von Emulgatoren eingesetzt werden, wobei beliebige im Stand der Technik bekannte Co-Emulgatoren oder Emulsionsstabilisatoren zugesetzt werden können.

Ein erfindungsgemäßes Gemisch weist einen mehr oder weniger intensiven Geruch nach Essig auf, der von einigen Verbrauchern als unangenehm empfunden wird. Zur Erhöhung der Verbraucher-Akzeptanz können deshalb Geruchsstoffe zugesetzt werden, wobei sowohl natürliche, als auch synthetische, im Stand der Technik zur Herstellung von Lebensmitteln und Kosmetika bekannte Geruchsstoffe in Betracht kommen.

Darüber hinaus können einem erfindungsgemäßen Gemisch ätherische Öle, wie beispielsweise Lavendelöl, Melissenöl, Zitronenöl, Orangenöl, Vanilleöl, Fichtennadelöl, Teebaumöl oder Kombinationen von ätherischen Ölen zugesetzt werden. Neben einer Überdeckung des Essiggeruches können die eingesetzten ätherischen Öle, die im Stand der Technik bekannten positiven Wirkungen von ätherischen Ölen aufweisen, und je nach beabsichtigter Anwendung gezielt ausgewählt werden.

Weiterhin können einem erfindungsgemäßen Gemisch beliebige weitere, im Stand der Technik zur Herstellung von Kosmetika oder Pharmazeutika, insbesondere von Cremes, Ölen und Badezusätzen, bekannte Inhaltsstoffe zugesetzt werden, d.h. beispielsweise beliebige Farbstoffe, Konservierungsmittel, Antioxidantien, Konsistenzgeber, Puffersubstanzen, Ölund Wasserphasenbestandteile, Antiseptika, Chelatbildner, Radikalfänger, Stabilisatoren, Aminosäuren, Kohlenhydrate, Vitamine, Pflanzenauszüge, Pflanzenpreßsäfte, anorganische Salze, Ethanol, Isopropanol, Dimethylsulfoxid, Verdicker, Glycerine, Quellstoffe, Tenside, Seifen, schäumende Substanzen, Schaumstabilisatoren oder Kombinationen hiervon.

Die vorliegende Erfindung betrifft weiter eine erfindungsgemäße Zubereitung umfassend 0,2 bis 3 Vol.-% an Essigsäure, bevorzugter 0,7 bis 2,4 Vol.-% an Essigsäure, 65 bis 90 Vol.-% an Öl, bevorzugter 75 bis 85 Vol.-% an Öl, und 5 bis 14 Vol.-% an Emulgator, bevorzugter 6 bis 12 Vol.-% an Emulgator. Alternativ können auch Zubereitungen umfassend 0,2 bis 3 Gew.-% an Weinsäure oder Zitronensäure, bevorzugter 0,7 bis 2,4 Gew.-% an Weinsäure oder Zitronensäure, 65 bis 90 Vol.-% an Öl, bevorzugter 75 bis 85 Vol.-% an Öl, und 5 bis 14 Vol.-% an Emulgator, bevorzugter 6 bis 12 Vol.-% an Emulgator oder Kombinationen (d.h. beispielsweise Gemische) von Essigsäure, Weinsäure oder Zitronensäure enthaltenden Zubereitungen verwendet werden.

Ein besonderer Vorteil einer erfindungsgemäßen Zubereitung ist, dass sie auf Basis weit verbreiteter Ausgangstoffe bei vergleichsweise geringen Kosten einfach hergestellt werden kann. Komplizierte, über mehrere Reaktionsstufen herzustellende synthetische Substanzen, die zudem von einigen Verbrauchern abgelehnt werden, müssen bei der vorliegenden Erfindung nicht zum Einsatz kommen. Eine erfindungsgemäße Zubereitung kann natürlich außer zur Behandlung von Menschen auch zur Behandlung von Tieren, insbesondere von Säugetieren dienen.

Die Erfindung wird anhand der folgenden, nicht einschränkenden Beispiele näher erläutert.

### Beispiel 1

### Johanniskraut-Avocado-Creme

50 ml Avocadoöl
50 ml Johanniskrautöl
20 ml Apfelessig
5 ml Vitamin E
5 ml Emulgator
20 g Bienenwachs

Die vorstehend aufgeführten Bestandteile werden unter Rühren im Wasserbad bis zum Schmelzen des Bienenwachses erwärmt und solange sorgfältig durchmischt bis eine Creme von gleichmäßiger Beschaffenheit erhalten wird. Optional werden anschließend 2 ml Lavendelöl untergemischt.

Die vorstehend angegebene Creme kann beispielsweise zum Einreiben ausgewählter Körperstellen oder großflächiger Körperbereiche verwendet werden.

### Beispiel 2

### Traubenkernöl-Creme

100 g Traubenkernöl
20 g Apfelessig
5 ml Vitamin E
5 ml Emulgator
20 g Bienenwachs

Die vorstehend aufgeführten Bestandteile werden unter Rühren im Wasserbad bis zum Schmelzen des Bienenwachses erwärmt und solange sorgfältig durchmischt bis eine Creme von gleichmäßiger Beschaffenheit erhalten wird. Optional werden anschließend 2 ml Lavendelöl untergemischt.

Die vorstehend angegebene Creme kann beispielsweise zum Einreiben ausgewählter Körperstellen oder großflächiger Körperbereiche verwendet werden.

### Beispiel 3

### Emulsin AS

50 ml Avocadoöl
50 ml Sojaöl
20 ml Essig
5 ml Vitamin E
5 ml Emulgator
2 ml Lavendelöl

Die vorstehend aufgeführten Bestandteile werden unter Rühren und/oder Schütteln miteinander vermischt.

Emulsin AS kann beispielsweise zum Einreiben ausgewählter Körperstellen oder großflächiger Körperbereiche oder als Badezusatz verwendet werden.

### Beispiel 4

### Emulsin JE

100 ml Johanniskrautöl
10 ml Essigessenz, Gehalt an Essigsäure: 25 %
5 ml Vitamin E
5 ml Emulgator
2 ml Lavendelöl

Die vorstehend aufgeführten Bestandteile werden unter Rühren und/oder Schütteln miteinander vermischt.

Emulsin JE kann beispielsweise zum Einreiben ausgewählter Körperstellen oder großflächiger Körperbereiche oder als Badezusatz verwendet werden.

### Beispiel 5

### Emulsin J

100 ml Johanniskrautöl
20 ml Essig
5 ml Tocopherol
5 ml Emulgator
2 ml Lavendelöl

Die vorstehend aufgeführten Bestandteile werden unter Rühren und/oder Schütteln miteinander vermischt.

Emulsin J kann beispielsweise zum Einreiben ausgewählter Körperstellen oder großflächiger Körperbereiche oder als Badezusatz verwendet werden.

### Beispiel 6

### Paraffin-Essig-Vitamin E-Zubereitung

20 ml dünnflüssiges Paraffin
20 ml dickflüssiges Paraffin
4 ml Tocopherol
4 ml Essigessenz, Gehalt an Essigsäure: 25 %
2 ml Vitamin E

Die vorstehend angegebene Paraffin-Essig-Vitamin E-Zubereitung kann beispielsweise zum Einreiben ausgewählter Körperstellen oder großflächiger Körperbereiche oder als Badezusatz verwendet werden.

### Beispiel 7

### Anwendungsergebnisse

Fünf unter Gicht leidende Personen rieben den Hautbereich im Bereich der betroffenen Gelenke einmal täglich während 10 Tagen mit Emulsin J (Beispiel 5) ein. Alle Personen berichteten von einer Abnahme der Schwellung im Bereich des betroffenen Gelenks und einer erheblichen Verringerung oder einem Verschwinden der Schmerzen.

Eine unter einer Sportverletzung am Knie leidende Person rieb den Hautbereich über dem Knie während einer Woche einmal täglich mit Emulsin AS (Beispiel 3) ein. Die Schwellung im Kniebereich und die Schmerzen dort gingen erheblich zurück.

Bei Patienten mit Venenentzündung und Thrombose wurde ein erfindungsgemäßes Gemisch äußerlich im Bereich der betroffenen Gliedmaßen aufgetragen. Hierbei wurde von einer kühlenden und schmerzlindernden Wirkung berichtet.

Bei einem männlichen Patienten mit einem amputationsgefährdeten Bein wurde das betroffene Bein an fünf aufeinander folgenden Tagen mit einem erfindungsgemäßen Gemisch massiert. Nach 5 Tagen konnte auf der Hautoberfläche das Auftreten einer gelben kristallinen oder kristall-ähnlichen Substanz beobachtet werden und nach weiteren zwei Wochen Behandlungsdauer berichtete der Patient, dass er ein besseres Gefühl im Bein habe.

Bei einer 76-jährigen Frau, die unter starken Kopfschmerzen litt wurde die Stirn mit einem erfindungsgemäßen Gemisch eingerieben. Hierbei konnte auf der Hautoberfläche die Bildung einer Sand-ähnlichen Substanz beobachtet werden. Nach dreimaligen Einreiben waren die Kopfschmerzen weitgehend verschwunden.

Bei einer 56-jährigen Frau, deren eine Körperseite, falls sie auf dieser lag, taub wurde, wurde der Rücken- und Nackenbereich dreimal mit einem erfindungsgemäßen Gemisch einmassiert. Die Frau berichtete von einem Verschwinden des Taubheitsgefühls.

Eine Frau litt unter einem Kribbeln und Kältegefühl in den Beinen, obwohl sich diese warm anfühlten. Nach täglichem Einreiben mit einem erfindungsgemäßen Gemisch der Beine während drei Wochen verschwanden Kältegefühl und Kribbeln.

Bei einem Mann wirkte eine Körpermassage mit einem erfindungsgemäßen Gemisch erektionsfördernd.

Bei einer Frau die unter zerebralen Durchblutungsstörungen litt, wurde im Rücken- und Nackenbereich ein erfindungsgemäßes Gemisch aufgetragen. Die Frau berichtete von einer Verringerung der Kopfschmerzen und einer Verbesserung ihrer Merkfähigkeit.

## Patentansprüche

1. Verwendung eines Gemisches umfassend Öl, Emulgator und mindestens eine organische Säure ausgewählt unter Essigsäure, Weinsäure, Zitronensäure zur Behandlung von Gelenkerkrankungen, zur Anregung der Durchblutung und zur Kühlung der Haut.

2. Verwendung eines Gemisches nach Anspruch 1, wobei das Gemisch zur Lockerung der Muskulatur, zur Verringerung eines Kältegefühls in den Gliedmaßen, zur Verringerung von Entzündungsreaktionen, zur Verbesserung des Zustands amputationsgefährdeter Gliedmaßen, zur Verringerung oder Behebung von Kopfschmerzen, bei Hämorrhoiden oder zur Erektionsförderung genutzt wird.

3. Verwendung eines Gemisches nach Anspruch 1, wobei die Gelenkerkrankungen ausgewählt sind unter Gicht, entzündlichen Gelenkerkrankungen, Überbeanspruchungen und rheumatischen Erkrankungen.

4. Verwendung eines Gemisches nach Anspruch 1 umfassend 0,2 bis 3 Vol.-% an Essigsäure, 65 bis 90 Vol.-% an Öl und 5 bis 14 Vol.-% an Emulgator, oder
umfassend 0,2 bis 3 Gew.-% an Weinsäure, 65 bis 90 Vol.-% an Öl und 5 bis 14 Vol.-% an Emulgator, oder
umfassend 0,2 bis 3 Gew.-% an Zitronensäure, 65 bis 90 Vol.-% an Öl und 5 bis 14 Vol.-% an Emulgator, oder
umfassend Kombinationen hiervon.

5. Verwendung nach einem der vorstehenden Ansprüche, wobei das Gemisch in Form einer Creme, eines Öls, eines Badezusatzes oder einer Formulierung zur oralen Einnahme vorliegt.

6. Verwendung nach einem der vorstehenden Ansprüche, wobei das Öl ein mineralisches Öl, ein pflanzliches Öl, ein tierisches Öl, ein Ölauszug pflanzlicher Bestandteile mit einem mineralischen, pflanzlichen oder tierischen Öl, ein Wachs und/oder Kombinationen hiervon ist.

7. Verwendung nach Anspruch 6, wobei das Öl ausgewählt ist aus der Gruppe bestehend aus Paraffinöl, Johanniskraut-Mazerat, Avocadoöl, Sojaöl, Schwarzkümmelöl, Ringelblumenöl, Traubenkernöl oder Kombinationen hiervon.

8. Verwendung nach einem der vorstehenden Ansprüche, wobei die organische Säure ausgewählt ist aus der Gruppe bestehend aus reiner Essigsäure, wässrigen Essigsäurelösungen, Essigessenz, Weinessig, Apfelessig, Weinsäure, wässrigen Weinsäurelösungen, Zitronensäure, wässrigen Zitronensäurelösungen oder Kombinationen hiervon.

9. Verwendung nach einem der vorstehenden Ansprüche, wobei der Emulgator ausgewählt ist aus der Gruppe bestehend aus Emulgatoren auf Basis von PEG - Derivaten, Vitamin E, Phospholipiden, Tocopherolen, Tocopherylacetaten, Tocopherol-Derivaten, Tocopherol-Vorläuferverbindungen und/oder aus Kombinationen hiervon.

10. Verwendung nach einem der vorstehenden Ansprüche, wobei das Gemisch weiter einen Geruchsstoff und/oder mindestens ein ätherisches Öl umfasst.

11. Zubereitung, umfassend 0,2 bis 3 Vol.-% an Essigsäure, 65 bis 90 Vol.-% an Öl und 5 bis 14 Vol.-% an Emulgator, oder
umfassend 0,2 bis 3 Gew.-% an Weinsäure, 65 bis 90 Vol.-% an Öl und 5 bis 14 Vol.-% an Emulgator, oder
umfassend 0,2 bis 3 Gew.-% an Zitronensäure, 65 bis 90 Vol.-% an Öl und 5 bis 14 Vol.-% an Emulgator, oder
umfassend Kombinationen hiervon.
